# EUROPEAN PATENT SPECIFICATION

(11) **EP 2 555 719 B1**
(45) Date of publication and mention of the grant of the patent: **02.03.2016**
(21) Application number: 10849565.6
(22) Date of filing: 06.04.2010
(51) Int. Cl.: A61F 5/01

(54) **A DEVICE FOR PROVIDING SUPPORT OF A FOOT**
VORRICHTUNG ZUR STÜTZE EINES FUSSES
DISPOSITIF DE SOUTIEN DU PIED

(43) Date of publication of application: 13.02.2013
(73) Proprietor: Camp Scandinavia AB, 254 67 Helsingborg (SE)
(72) Inventor: NORDSTRÖM, Kjell, S-722 40 Västerås (SE)
(74) Representative: Awapatent AB
(86) International application number: PCT/SE2010/050379
(87) International publication number: WO 2011/126415

(56) References cited:
- WO-A1-88/00033
- WO-A1-2006/010213
- CH-A5- 593 057
- FR-A- 731 133
- US-A- 1 402 282
- US-A- 5 291 904
- US-A- 5 291 904
- US-A1- 2008 077 066
- US-A1- 2008 077 066
- US-A1- 2009 227 928

## Description

### Technical field

The present invention relates to a device for providing support of a foot comprising a rail and a strap, the rail having a longitudinal extension to be arranged along the lower leg of a user, and the strap being arranged to extend from a connection point on said rail to the foot of the user.

### Background

Many persons are suffering from problems to walk as a result of medical conditions affecting one or both foot/feet, such as the medical condition known as drop foot. The problems to walk may be the result of reduced lift of the foot/feet and thus risk of tripping during walking.

It can also be the result of injuries to ligaments in the foot. It can be the result of problems such as pronation, or supination or any combination of drop foot, pronation, supination, and/or plantar fasciitis.

Disclosures of devices providing lift to the foot during walking exist, for example the US Patent application with publication number US 2008/0077066 disclosing an ankle foot orthosis for patients with drop foot.

Another example is US2009/0227928 upon which the preamble of claim 1 is based.

### Summary of the invention

It is an object of the present invention to make available an efficient device for providing support of a foot.

Another object is to provide a device that is easy to mount and adjust to different persons individual requirements and needs, and for providing support to a foot/feet of the persons suffering from conditions such as drop foot, pronation, supination or plantar fasciitis, or any combination of drop foot, pronation, and/or supination, or any condition where dorsal flexion of the foot may be requested.

The present invention relates to a device for providing support of a foot as claimed in claim 1.

The device forms an efficient means for rehabilitation curing, relieving or preventing medical conditions associated with reduced or abnormal lift of the foot during walking, and/or rehabilitating from such medical conditions. The device may also be used for stretching of the dorsal and plantar structures such as soft tissue, ligaments, tendons, and muscles.

The strap efficiently provides for a lifting force acting on the foot, whereby the risk of tripping during walking with the device may be reduced, minimized or avoided.

The at least one guiding means is efficiently used to guide the strap and to hold it close to the lower leg, the ankle, and/or the foot of the of the user of the device, whereby the risk of the strap being caught by objects in the way of the walking path may be reduced, minimized or avoided. Thus, the risk of tripping during walking may be reduced. The guiding means further facilitates or simplifies the wearing of for example trousers or a skirt in combination with the device. Not only is the strap less visible but also less subjected to wear.

The rail having a longitudinal extension to be arranged along the lower leg of a user may facilitate a long strap to be used and facilitates that the force acting on the lower leg of the user is divided over a large area of said lower leg. This improves the comfort of the wearer.

By said first portion of the strap being made of a resilient material a lifting force of the leg is efficiently conveyed to the foot even when/if the foot is angled upwards, during for example walking. It also efficiently enables the foot to be angled downwards.

By said second portion of said strap being made of a rigid material wear of the second portion of said strap may be reduced. Further, friction may be reduced between the second portion of said strap and any part being in contact with said second portion of said strap. This can be for example the lower end of a trouser leg.

The term resilient material as used herein refers to a material being stretchable. The resilient material has spring back properties when stretched. The resilient material may thereby provide a lifting force for the foot of the user of the device according to the invention. The resilient material may, for example, be selected from the group comprising rubber, polymer, plastic material, springs, or combinations thereof. The resilient material may comprise rubber.

The term rigid material as used herein refers to any material suitable for the invention, which material being essentially not stretchable, Particularly, the rigid material is essentially not strechable or resilient in the longitudinal direction of the strap. The rigid material may though be bendable. Such a material may be, for example, a woven or braided material, a fabric, leather, a polymeric material, such as a plastic or a rubber material, or a metal.

Accordingly, by said combination of features the inventive device may provide support for a foot of a person suffering from drop foot, pronation, supination, and/or plantar fasciltis, or any combination thereof.

Said first portion of said strap may be attachable to said connection point on said rail, and said second portion of said strap may be attachable to said connection means, the connection means being adapted to be arranged on the foot of the user,

Said second portion of said strap may be attachable to said connection point on said rail, and said first portion of said strap may be attachable to said connection means, the connection means being adapted to be arranged on the foot of the user.

Said second portion of said strap may be passing said guiding means. Thus, wear caused by friction between said guiding means and the strap may be directed towards the second portion of said strap. Thus, any wear to the first portion of said strap, caused by friction between said guiding means and the strap, may be reduced, minimised or avoided. For the case when the first portion of said strap which second portion of said strap may be manufactured from wear resistant material, thus little affected by the friction and wear.

It is realised that it may be that in a normal case or during normal use said second portion of said strap is passing through said guiding means and in extreme cases the first portion may pass through the guiding means if the foot is bent downwards more than in a normal case.

The position of said guiding means may be adjustable along and/or transverse the longitudinal extension of the rail. Thus, the degree of stretching of the strap, or the resilient first part of the strap, may be adjusted by adjusting said guiding means. Thus the lifting force to the foot may be adjusted. Adjusting the position of the guiding means along the longitudinal extension of the rail in a direction towards or from the knee of the user of the device may change the degree of stretching of the strap, and thus the lifting force to the foot. Accordingly, the strap may be adjusted to minimise the risk of tripping during walking.

Adjusting the position of the guiding means transverse the longitudinal extension of the rail may adjust the lifting force towards one side of the foot. Thus, the device may efficiently be used for treatment or as aid for persons suffering from, for example, pronation, supination, or any combination of drop foot, pronation, supination and/or plantar fasciitis. The device, thus, may assist in maintaining the foot in a neutral position.

The position of said connection point on said rail may be adjustable along the longitudinal extension of the rail, thereby allowing easy and efficient adjustment of the stretch of said fist portion of said strap. This may be advantageous, for example, for adjusting the lifting force to the foot to suit the need of lifting force to the foot. For example, the lifting force may be adjusted as a result of the medical condition being improved or worse. The user of the device may also adjust the lifting force to the foot, for example, as the result of the user feeling the need of lesser lifting force in the beginning of a day and greater lifting force at the end of a day, or *vice versa*. Adjustments to the lifting force may also be advantageous if the user changes foot wear such that e.g. the weight or design of the shoe changes or if the device is used by several users in need of different lifting forces. Further, the adjustments to the lifting force may enable manufacturing of one type of device suitable for several persons.

Said first portion of said strap may be arranged to extend essentially parallel to the longitudinal extension of said rail.

Thus, the risk of the strap being caught by objects in the way of the walking path, such as object on the ground e.g. branches, may be reduced, minimized or avoided. Further, it may facilitate or simplify the wearing of for example trousers or a skirt in combination with the wearing of the device. Also, it may reduce, minimize or avoid any visible evidence of the strap, and particularly if the device is worn in combination with for example trousers or a skirt.

Said connection point may be releasably attachable to the rail. This can be made by any suitable means, for example means selected from the group comprising Velcro, snap fastener, buttons, or any combinations thereof.

Thus, one strap may efficiently be replaced by another strap, for example if the strap is defect, or if another strap with different properties than the one strap is desired.

Said connection means may be selected from the group comprising a hook, a loop, a spring hook, Velcro, button, and snap fastener, or any combination thereof. Thus, the connection means may efficiently be arranged to, for example, a foot wear of a user of the device, or directly to the foot of a user of the device.

The device may comprise an upper strapping means, and a lower strapping means. Thus the device may efficiently be positioned on the lower leg of a user of the device.

Said guiding means may be selected from the group comprising a pulley, a roller, a hook, a loop, a spring hook and a feed track, or any combination thereof. Thus, said guiding means may efficiently be guiding said strap. This can be made with little friction between said guiding means and said strap.

Said connection means may be arranged to be connected to a foot-wear or directly to the foot of the user. Said foot wear may be selected from the group comprising shoes, sandals, socks, casting boots, boots, orthoses as such, foot orthoses, casts, straps, insoles, slippers, and ankle-foot orthoses, or any combination thereof.

Said guiding means may be made from rigid material. It is preferred that the guiding means exhibits rigidity at least in the direction in which the strap applies a force to the guiding means during normal use of the device. Thus, efficient guiding of the strap may be provided, and low friction may be obtained between said guiding means and said strap.

### Brief description of the drawings

Figure 1 illustrates a device according to one embodiment of the invention.
Figures 2a and 2b illustrates a parts of a device according to one embodiment of the invention as positioned on a lower leg of a user wearing a shoe.
Figure 2c illustrates a part of a lower leg and a shoe.

### Detailed description

The invention will now be explained in more detail, and specific preferred embodiments, and variations of these, will be shown. The explanations are intended for illustrative and explanatory purposes and are not to be seen in any way as limiting the scope of the invention. The illustrations are schematic and all details are not illustrated, and it is to be understood that all illustrated details may not be necessary for the invention.

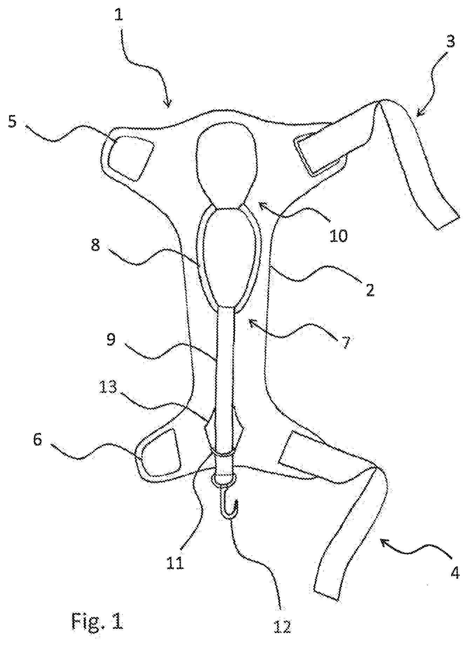

A device 1 for providing support of a foot according to one embodiment of the invention is illustrated in figure 1.

The device 1 has a rail 2. In this particular example, the rail 2 is made of plastic material, which plastic material is bendable, such that it may be shaped to follow the lower leg of a user of the device 1. Although not seen in the figure, it may be preferred that the side of the rail 2 intended to be facing the leg during use may be covered by a soft and/or cushioning material, such as foamed plastic, thick fabric or felt to make the device 1 comfortable to the wearer. Such a soft and/or cushioning material may be releasably attachable by any suitable means, for example Velcro.

The rail can be releasably attached to a lower leg of the user of the device by means of the upper strapping means 3 and the lower strapping means 4.

The strapping means 3 and 4 are, according to this particular embodiment, ribbons made from fabric. The strapping means 3 and 4 can have a surface structure which enables them to be attached to the Velcro 5, 6 attached to the rail 2. The strapping means 3 and 4 might also have had attached piece(s) of Velcro corresponding to the Velcro 5, 6. Other means suitable for the purpose of releasably attaching the strapping means 3 and 4 to the rail 2 may be used, for example means selected from the group comprising, a hook, a button, Velcro, and snap fastener, or any combination thereof.

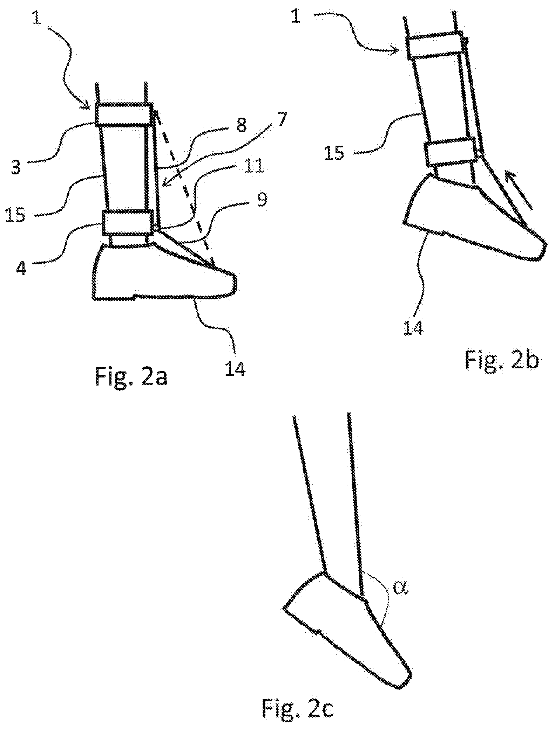

When the device 1 is attached to the lower leg, the rail 2 would be positioned in contact with the front of the lower leg, and the strapping means 3 and 4 would be wrapped behind and around the lower leg and attached to the rail 2, thus securing the device 1 to the leg. In the disclosed embodiment, the upper strapping means 3 is arranged to be connected in the region of the calf of the user, and the lower strapping means 4 is arranged to be connected proximal to the ankle of the user and distal to the calf of the user, see figure 2a.

The upper strapping means is attached to a piece of Velcro 5 of the rail 2, and the lower strapping means is attached to another piece of Velcro 6. The strapping means 3 and 4 as such can be attached at their other ends to the rail 2 by means of Velcro.

It is realized that the strapping means 3 and 4 at one or both ends may be attached by other means to the rail 2, such as by means of buttons or they may be sewn to the rail 2, or a combination thereof. It is further realised that the strapping means may be of other types than ribbons. The at least one strapping means may, for example, be a clamp, a loop, or a sock or part of a sock.

The device 1 in figure 1 further has a strap 7, which strap 7 has a first portion 8 and a second portion 9. The first portion 8 of said strap 7 is made from a resilient material, in this particular example being a bundle of rubber threads, which bundle is covered by stretchable fabric. It is realised that the first portion 8 of said strap 7 may not have the shape of a loop as in this particular example, but rather any longitudinal shape. In figure 1 the strap 7 is illustrated relaxed, or not stretched.

The second portion 9 of said strap 7 is made of a rigid material, in this particular example being a ribbon of fabric which is bendable but essentially not stretchable. It is realised that also the second portion 9 of said strap 7 can have any longitudinal shape.

The first portion 8 of said strap is attached to the rail 1 at a connection point 10 by means of Velcro. The Velcro facilitates that the attachment point may be moved, for example, up or down along the longitudinal extension of the rail 2, thus facilitating adjustments to the stretch of the strap 7 and thus the lift to the foot of the user of the device 1. It can of course also be moved in the transverse direction of the rail.

As seen in figure 1, the first portion 8 of the strap 7 is arranged extending essentially parallel to the longitudinal extension of said rail 2.

The second portion 9 of said strap 7 is passing through a guiding means 11. In the disclosed embodiment, the guiding means is a D-shaped loop with smooth edges made from metal. Smooth edges result in reduced wear to the strap 7, as compared with for example a loop with rough edges. It is to be understood that this is only one out of many possible shapes and types of a guiding means.

In the disclosed embodiment, the guiding means 11 is arranged on the rail 2 by means of a piece of Velcro 13 attached to the loop and a corresponding piece of Velcro (not disclosed) attached to the rail 2. Thus, the position of then guiding means 11 may be adjustable along and/or transverse the longitudinal extension of the rail. Thereby, the guiding means 11 may be adjustable along the longitudinal extension of the rail. Further, the guiding means 11 may be adjustable transverse the longitudinal extension of the rail.

The second portion of the strap 9 is passing the guiding means 11, thus minimising the risk of wear to the resilient first portion of said strap, and the strap 7 extends to a connection means 12 in the form of a hook. The connection means 12 is in the disclosed embodiment arranged in the lower free end of the strap 7.

When the device is in use, the connection means 12 will be arranged to the foot of the user of the device. The connection means 12 in this example may, for example, be hooked to the shoe strings of the shoe of the user of the device. The connection means 12 can have any other suitable shape. For example, it can have the shape of a loop to be thread over the foot or a toe of the user of the device. For example, said connection means 12 may be selected from the group comprising a hook, a loop, a spring hook, a button, Velcro, and snap fastener, or any combination thereof.

If arranged to a foot of a user, the strap 7 would be stretched due to the resilient first portion 8 of said strap 7, and the device 1 would thus provide a lifting force acting on the foot of a user of the device.

According to one embodiment of the invention (not disclosed), the rail 2 may be telescopic. Thus, a single device may be used by users with different sizes of their legs.

The rail 2 may have a concave cross-section, the concave surface facing the leg of the user during use of the device.

The rail 2 may also, for example in accordance with the example discussed above and illustrated by figure 1 be made of a bendable material allowing the rail 2 to be shaped according to the shape of the leg of the user of the device.

With reference to figures 2a and 2b, use of the device will be illustrated according to one embodiment of the invention.

Figures 2a and 2b are schematic illustrations of parts of the device according to the invention of figure 1. Note that to improve the clarity of figures 2a and 2b, all parts of the device are not illustrated, but only selected parts.

Figure 2a illustrates a shoe 14, and a part of a lower leg 15 of the user of the device 1. Of the device 1, the upper strapping means 3, the lower strapping means 4, the guiding means 11, and the strap 7 are illustrated. Note that, for example, the rail is not illustrated. The second portion of said strap 9 is passing through the guiding means 11, while the first part of said strap 8 does not. The connection means or how it is attached to the shoe is not illustrated, but it is clear from figures 2a and 2b, that the connection means is attached in the frontal part of the shoe 14. Figure 2a illustrates part of the leg and use of the user when standing in an upright position. The sole of the shoe 14 is resting on the ground.

The dotted line in figure 2a illustrates how the strap 7 would have extended, had the strap 7 not been passing said guiding means 11. It is evident from figure 2a that the guiding means according to the invention minimises the risk of the strap 7 getting entangled or stuck to objects during walking, as it reduces the gap between the strap 7 and the leg 15, the ankle, and the shoe14, as compared to any strap following the dotted line..

Figure 2b illustrates the same part of the leg 15, shoe 14, and device 1 when the shoe is lifted from the ground, for example when taking a step during walking. As the shoe 14 left the ground, the shoe and foot will be experience a force from gravity downwards towards the ground. The strap, and particularly when the first part of said strap 8 being resilient is stretched out, will lift the foot upwards opposite the force from gravity, in the direction indicated by the arrow in figure 2b. Thus, the risk of tripping by will be reduced, minimised, or avoided during walking. Figure 2c illustrates, schematically, that an angle α between the shoe 14 and the leg 15 is considerably greater if the device 1 and the resulting lifting from the strap 7 is not present and the risk of tripping is greater.

It is realised that the angle between the shoe 14 and the leg 15 in figure 2b may be altered by adjusting the stretch of the first part 8 of said strap 7, for example by the procedures exemplified above in the discussions concerning figure 1, for example by moving the connecting points and/or guiding means in the longitudinal directions and/or transverse directions of the rail.

The first part 8 of said strap 7 being resilient is further beneficial as it enables the foot to be bent downwards towards the ground. Thus, the strap 7 according to the invention may enable bending the foot downwards and assisting in bending the foot upwards. Thus, said first portion of the strap may be kept far away from the ground during for example walking, thus subjected to little or no wear. The second portion may be arranged in the proximity of the foot and thus subjected to more stress during walking compared to the first portion of said strap, for example stress from objects on the ground or from the shoe 14. Therefore, the second portion 9 of said strap 7 may be made from more wear-resistant material than the first part 8 of said strap 9 is made from.

The description given above relates to a number of embodiments. It should be understood that modifications thereto are possible, whereby the scope of protection should be determined by that of the claims.

## Claims

1. A device (1) for providing support of a foot, comprising
a rail (2), at least one strapping means (3, 4), at least one guiding means (11), and a strap (7), wherein
said rail (2) having a longitudinal extension to be arranged along the lower leg of a user, and said rail (2) being releasably attachable to said lower leg by said at least one strapping means (3, 4),
said strap (7) being arranged to extend from a connection point (10) on said rail via said at least one guiding means (11) arranged on said rail (2), to a connection means (12) which is adapted to be arranged to the foot of the user,
said strap (7) having a first portion (8) and a second portion (9), the second portion (9) passing through the guiding means (11) **characterised in that** said first portion (8) forms a first element which is made of a resilient material, and said second portion (9) forms a second element which is made of a rigid material.

2. The device (1) according to claim 1, wherein said first portion (8) of said strap (7) is attachable to said connection point (10) on said rail (2), and said second portion (9) of said strap (7) is attachable to said connection means (12), the connection means (12) being adapted to be arranged on the foot of the user.

3. The device (1) according to claim 1, wherein said second portion (9) of said strap (7) is attachable to said connection point (10) on said rail (2), and said first portion (8) of said strap (7) is attachable to said connection means (12), the connection means (12) being adapted to be arranged on the foot of the user.

4. The device (1) according to anyone of the previous claim, wherein the position of said guiding means (11) is adjustable along and/or transverse the longitudinal extension of the rail (2).

5. The device (1) according to anyone of the previous claims, wherein the position of said connection point (10) on said rail (2) is adjustable along the longitudinal extension of the rail (2), thereby allowing adjustment of the stretch of said first portion (8).

6. The device (1) according to anyone of the previous claims, wherein said first portion (8) is arranged to extend essentially parallel to the longitudinal extension of said rail (2).

7. The device (1) according to anyone of the previous claims, wherein said connection means (12) is selected from the group comprising a hook, a loop, a spring hook, a button, Velcro, and snap fastener, or any combination thereof.

8. The device (1) according to anyone of the previous claims, wherein said at least one strapping means (3,4) comprises an upper strapping means (3), and a lower strapping means (4).

9. The device (1) according to anyone of the previous claims, wherein said guiding means (11) is selected from the group comprising a pulley, a roller, a hook, a loop, a spring hook and a feed track, or any combination thereof.

10. The device (1) according to anyone of the previous claims, wherein said connection means (12) is adapted to be connected to a foot-wear or directly to the foot of the user.

11. The device (1) according to anyone of the previous claims, wherein said guiding means (11) is made from rigid material.

## Patentansprüche

1. Vorrichtung (1) zur Stütze eines Fußes, welche Folgendes umfasst
eine Schiene (2), mindestens ein Umschnürungsmittel (3, 4), mindestens ein Führungsmittel (11) und ein Spannband (7), wobei
die Schiene (2) eine längslaufende Verlängerung aufweist, die entlang des Unterschenkels eines Benutzers angeordnet wird, und die Schiene (2) durch das mindestens eine Umschnürungsmittel (3, 4) lösbar an dem Unterschenkel befestigt werden kann,
wobei das Spannband (7) derart angeordnet ist, dass es sich von einem Verbindungspunkt (10) an der Schiene über das mindestens eine Führungsmittel (11), das an der Schiene (2) angeordnet ist, zu einem Verbindungsmittel (12), das am Fuß des Benutzers angeordnet werden kann, erstreckt,
wobei das Spannband (7) einen ersten Abschnitt (8) und einen zweiten Abschnitt (9) aufweist, wobei der zweite Abschnitt (9) durch das Führungsmittel (11) verläuft, **dadurch gekennzeichnet, dass** der erste Abschnitt (8) ein erstes Element bildet, welches aus einem elastischen Material hergestellt ist, und der zweite Abschnitt (9) ein zweites Element bildet, welches aus einem steifen Material hergestellt ist.

2. Vorrichtung (1) nach Anspruch 1, wobei der erste Abschnitt (8) des Spannbandes (7) an dem Verbindungspunkt (10) an der Schiene (2) befestigt werden kann und der zweite Abschnitt (9) des Spannbandes (7) an dem Verbindungsmittel (12) befestigt werden kann, wobei das Verbindungsmittel (12) am Fuß des Benutzers angeordnet werden kann.

3. Vorrichtung (1) nach Anspruch 1, wobei der zweite Abschnitt (9) des Spannbandes (7) an dem Verbindungspunkt (10) an der Schiene (2) befestigt werden kann und der erste Abschnitt (8) des Spannbandes (7) an dem Verbindungsmittel (12) befestigt werden kann, wobei das Verbindungsmittel (12) am Fuß des Benutzers angeordnet werden kann.

4. Vorrichtung (1) nach einem der vorhergehenden Ansprüche, wobei die Position des Führungsmittels (11) entlang und/oder quer zu der längslaufenden Verlängerung der Schiene (2) angepasst werden kann.

5. Vorrichtung (1) nach einem der vorhergehenden Ansprüche, wobei die Position des Verbindungspunktes (10) an der Schiene (2) entlang der längslaufenden Verlängerung der Schiene (2) angepasst werden kann, wodurch eine Anpassung der elastischen Dehnung des ersten Abschnittes (8) gestattet wird.

6. Vorrichtung (1) nach einem der vorhergehenden Ansprüche, wobei der erste Abschnitt (8) derart angeordnet ist, dass er sich im Wesentlichen parallel zu der längslaufenden Verlängerung der Schiene (2) erstreckt.

7. Vorrichtung (1) nach einem der vorhergehenden Ansprüche, wobei das Verbindungsmittel (12) ausgewählt ist aus der Gruppe umfassend einen Haken, eine Schlaufe, einen Federhaken, einen Knopf, einen Klettverschluss und einen Druckknopf oder jegliche Kombination davon.

8. Vorrichtung (1) nach einem der vorhergehenden Ansprüche, wobei das mindestens eine Umschnürungsmittel (3, 4) ein oberes Umschnürungsmittel (3) und ein unteres Umschnürungsmittel (4) umfasst.

9. Vorrichtung (1) nach einem der vorhergehenden Ansprüche, wobei das Führungsmittel (11) ausgewählt ist aus der Gruppe umfassend eine Rolle, eine Walze, einen Haken, eine Schlaufe, einen Federhaken und eine Zuführbahn oder jegliche Kombination davon.

10. Vorrichtung (1) nach einem der vorhergehenden Ansprüche, wobei das Verbindungsmittel (12) an Schuhwerk angebracht oder direkt mit dem Fuß des Benutzers verbunden werden kann.

11. Vorrichtung (1) nach einem der vorherigen Ansprüche, wobei das Führungsmittel (11) aus steifem Material hergestellt ist.

## Revendications

1. Dispositif (1) destiné à supporter un pied, comprenant
un rail (2), au moins un moyen de fixation (3, 4), au moins un moyen de guidage (11) et une bride (7), dans lequel
ledit rail (2) ayant une extension longitudinale est conçu pour être disposé le long du bas de la jambe d'un utilisateur et ledit rail (2) peut être attaché de manière dissociable audit bas de jambe par ledit au moins un moyen de fixation (3, 4),
ladite bride (7) étant conçue pour s'étendre depuis un point de connexion (10) sur ledit rail via ledit au moins un moyen de guidage (11) disposé sur ledit rail (2) jusqu'à un moyen de connexion (12) qui est apte à être disposé sur le pied de l'utilisateur,
ladite bride (7) possédant une première partie (8) et une seconde partie (9), la seconde partie (9) passant à travers le moyen de guidage (11), **caractérisé en ce que** ladite première partie (8) forme un premier élément qui est en matériau résilient et ladite seconde partie (9) forme un second élément qui est en matériau rigide.

2. Dispositif (1) selon la revendication 1, dans lequel ladite première partie (8) de ladite fixation (7) peut être attachée audit point de connexion (10) sur ledit rail (2) et ladite seconde partie (9) de ladite fixation (7) peut être attachée audit moyen de connexion (12), le moyen de connexion (12) étant apte à être disposé sur le pied de l'utilisateur.

3. Dispositif (1) selon la revendication 1, dans lequel ladite seconde partie (9) de ladite bride (7) peut être attachée audit point de connexion (10) sur ledit rail (2) et ladite première partie (8) de ladite bride (7) peut être attachée audit moyen de connexion (12), le moyen de connexion (12) étant apte à être disposé sur le pied de l'utilisateur.

4. Dispositif (1) selon l'une quelconque des revendications précédentes, dans lequel la position dudit moyen de guidage (11) est réglable le long et/ou transversalement à l'extension longitudinale du rail.

5. Dispositif (1) selon l'une quelconque des revendications précédentes, dans lequel la position dudit point de connexion (10) sur ledit rail (2) est réglable le long de l'extension longitudinale du rail (2), ce qui permet le réglage de l'étirement de ladite première partie (8).

6. Dispositif (1) selon l'une quelconque des revendications précédentes, dans lequel ladite première partie (8) est disposée de manière à s'étendre sensiblement à la parallèle du sens longitudinal dudit rail (2).

7. Dispositif (1) selon l'une quelconque des revendications précédentes, dans lequel ledit moyen de connexion (12) est sélectionné dans le groupe comprenant un crochet, une boucle, un crochet à ressort, un bouton, du Velcro et un bouton pression ou toute combinaison de ceux-ci.

8. Dispositif (1) selon l'une quelconque des revendications précédentes, dans lequel ledit au moins un moyen de fixation (3, 4) comprend un moyen de fixation supérieur (3) et un moyen de fixation inférieur (4).

9. Dispositif (1) selon l'une quelconque des revendications précédentes, dans lequel ledit moyen de guidage (11) est sélectionné dans le groupe comprenant une poulie, une roulette, un crochet, une boucle, un crochet à ressort et une piste d'entraînement ou toute combinaison de ceux-ci.

10. Dispositif (1) selon l'une quelconque des revendications précédentes, dans lequel ledit moyen de connexion (12) est apte à être connecté à un article chaussant ou directement au pied de l'utilisateur.

11. Dispositif (1) selon l'une quelconque des revendications précédentes, dans lequel ledit moyen de guidage (11) est en matériau rigide.
